# EUROPEAN PATENT APPLICATION

(11) **EP 1 777 224 A2**
(43) Date of publication of application: **25.04.2007**
(21) Application number: 06021532.4
(22) Date of filing: 13.10.2006
(51) Int. Cl.: C07D 403/10, C07D 257/04

(54) **A process for the preparation of angiotensin II antagonistic compounds**

(30) Priority: 20.10.2005 IT MI20051989
(71) Applicant: Dipharma S.p.A., 33036 Mereto di Tomba (Udine) (IT)
(72) Inventor: Allegrini, Pietro, 20097 S.Donato Milanese (MI) (IT); Razzetti, Gabriele, 20099 Sesto S.Giovanni (MI) (IT); Rossi, Roberto, 27100 Pavia (IT); Lucchini, Vittorio, 20097 S.Donato Milanese (MI) (IT); Mantegazza, Simone, 20144 Milano (IT); Rasparini, Marcello, 27100 Pavia (IT)
(74) Representative: Minoja, Fabrizio

(57) **Abstract**

A process for the preparation of angiotensin II antagonists and novel intermediates useful for the synthesis thereof.

## Description

### FIELD OF THE INVENTION

The present invention relates to a process for the preparation of angiotensin II antagonists and novel intermediates useful for the preparation thereof.

### TECHNOLOGICAL BACKGROUND

Angiotensin II antagonists are medicaments useful in the treatment of hypertension, anxiety, glaucoma and heart attacks. Many of these compounds share a characteristic biphenyltetrazole moiety and have the following formula (**I**) 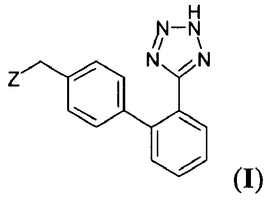 wherein Z is an optionally substituted heterocycle containing at least one nitrogen atom; or an open amide residue.

A number of processes for the preparation of compounds of formula **(I)** are known, in which the tetrazole and/or the carboxylic functionalities are protected in one of the starting synthetic steps, and then deprotected in one of the final steps. By way of example, R.D. Larsen et al. in J. Org. Chem. 1994, 59, 6391-6394 disclose the preparation of losartan through a cross coupling reaction between a compound of formula **(A)** 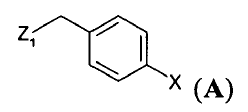 wherein Z₁ is 2-butyl-4-chloro-5-hydroxymethyl-imidazol-1-yl; X is bromine;
and a synthon of formula (**B**) 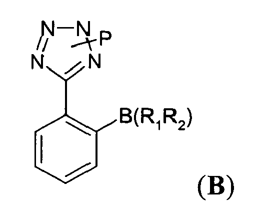 wherein -B(R₁R₂) is a disubstituted boron atom; P is a protective group;
and the subsequent removal of the protective group.

According to WO 2004/065383, irbesartan can be obtained analogously by reacting a compound of formula (**A**) in which Z1 is [2-butyl-1,3-diazaspiro[4,4]non-1-en-4-on-3-yl] and X is bromine, with a synthon of formula (**B**) wherein P and -B(R₁R₂) are as defined above. Valsartan can be obtained, for example, by tetrazolization of the respective nitrile or by acylation of the respective amine with valeroyl chloride, as disclosed in EP 443 983; or according to the processes described e.g. in WO 04/026847 and US 2005 059827. Also according to these documents, the tetrazole and/or the carboxylic functions are protected in one of the starting synthetic steps and then deprotected in one of the final steps. WO 2005/7102987, published on 3 November 2005, describes the synthesis of valsartan, without racemisation problems, comprising the formation of a biphenyl system in the presence of a palladium catalyst and is a solvent medium chosen from an aqueous or polar organic solvent, or a mixture of water and water-miscible solvent.

The protection/deprotection of the functional groups is an usual technique in organic synthesis for increasing selectivity to the desired product, avoiding the formation of by-products. From an industrial point of view this requires however addition procedures, which means longer times and increased costs.

It has now been found that the above mentioned cross coupling reaction can surprisingly be carried out in good yields also in the absence of protective groups for the carboxylic and/or tetrazole functions, thereby making the synthesis of the compounds of formula **(I)** much more advantageous.

### DETAILED DISCLOSURE OF THE INVENTION

The present invention relates to a process for the preparation of a compound having formula **(I),** or a salt thereof 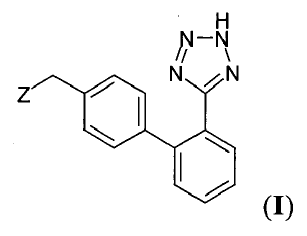 wherein
Z is an optionally substituted heterocycle containing at least one nitrogen atom, or an open amide residue;
which comprises
reacting a compound of formula **(II),** or a salt thereof 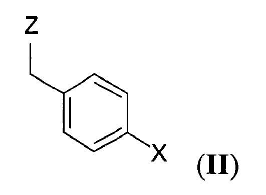 wherein
X is a leaving group and Z is as defined above;
with a synthon of formula **(III),** or a salt thereof 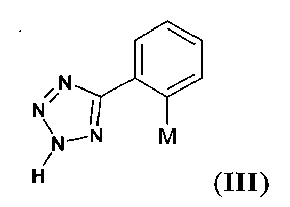 wherein
M is a -B(OR₁OR₂) group wherein
each of R₁ and R₂ is independently is hydrogen, C₁-C₈ alkyl, aryl, aryl-C₁-C₈ alkyl, or
R₁ and R₂, taken together, form a -(CH₂)ₘ-V-(CH₂)ₙ- group,
wherein
m and n, which can be the same or different, are 0 or 1, and
V is NR₃ or C(R₃)₂
wherein R₃ is hydrogen, C₁-C₈ alkyl, aryl or aryl-C₁-C₈ alkyl; or
M is a lithium or copper atom or a halogenated metal;
in the presence of a catalyst, an organic ligand and a basic agent and, if desired, converting a compound of formula **(I)** to a salt thereof and/or, if desired, converting a salt of a compound of formula **(I)** into its free acid; and wherein when, at the same time, Z is the amide residue (S)-N-(1-carboxy-2-methylprop-1-yl)-N-pentanoylamino, X is halogen or an -OSO₃R group, in which R is CF₃, tosyl, mesyl or F; M is a -B(OR₁OR₂) group wherein each of R₁ and R₂ is hydrogen, and the catalyst is a palladium compound, then the reaction is carried out in a solvent other than an aqueous or polar organic solvent, or a mixture of water and water-miscible solvent.

More particularly, substituent Z can be one of the following residues 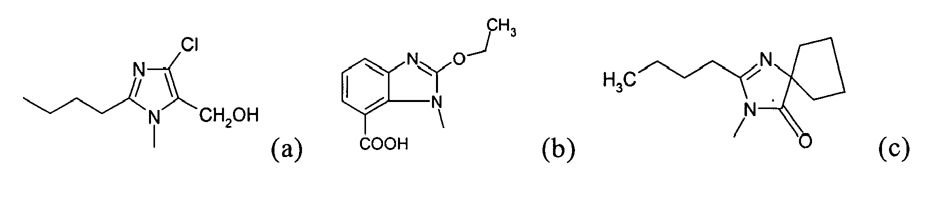 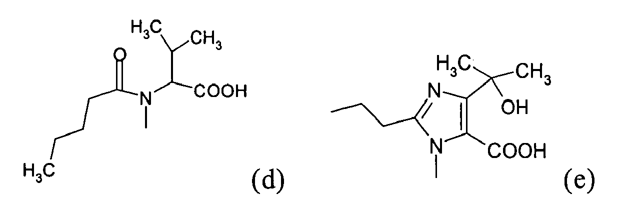 which identify specific angiotensin II antagonists:
a) [2-butyl-4-chloro-5-hydroxymethyl-imidazol-1-yl]: losartan;
b) [2-ethoxy-3H-benzoimidazole-4-carboxylic acid]: candesartan;
c) [2-butyl-1,3-diaza-spiro[4,4]non-1-en-4-on-3-yl]: irbesartan;
d) [(S)-N-(1-carboxy-2-methylprop-1-yl)-N-pentanoylamino]: valsartan; and
e) [5-carboxy-4-(1-hydroxy-1-methylethyl)-2-propyl-imidazo-1-yl]: olmesartan.

A salt of a compound of formula **(I), (II)** or **(III)** can be, for example, a pharmaceutically acceptable salt, such as the sodium, potassium, magnesium, calcium, zinc or copper salt, particularly the sodium or potassium salt.

A leaving group X is typically a halogen atom, for example chlorine, bromine or iodine, in particular bromine; or a hydroxy group activated through esterification, for example with an alkanesulfonate group, typically methanesulfonyloxy, toluenesulfonyloxy, fluorosulfonyloxy, trifluoromethanesulfonyloxy or nonafluorobutanesulfonyloxy. Preferably, the leaving group X is a halogen atom, in particular bromine.

M as a halogenated metal is, for example, a zinc, magnesium, nickel, copper or boron halide, preferably -ZnCl, -MgCl, -NiCl, -CuCl, -BCl₂, -ZnBr, -MgBr, -CuBr, and -BBr₂, more preferably ZnCl.

In an M, R₁, R₂ and/or R₃ group, a C₁-C₈ alkyl group or residue, which can be straight or branched, is preferably a C₁-C₄ alkyl, in particular methyl, ethyl, propyl, isopropyl, butyl, sec-butyl or tert-butyl.

In an M, R₁, R₂ and/or R₃ group, an aryl group is for example phenyl or naphthyl, preferably phenyl.

In an M, R₁, R₂ and/or R₃ group, an aryl-C₁-C₈ alkyl group is preferably a benzyl or phenylethyl group.

M is preferably a -B(OR₁OR₂) group wherein each of R₁ and R₂ is, independently, hydrogen or C₁-C₄ alkyl, in particular hydrogen.

A catalyst is typically a Pd, Pt or Ni salt, such as a chloride, bromide, iodide, acetate, acetyl acetonate, carbonate, hydroxide, preferably a palladium (II) salt, in particular palladium (II) acetate.

An organic ligand is typically a phosphine, such as tricyclohexyl phosphine, triphenylphosphine, tris-(3-hydroxypropyl)-phosphine, tributylphosphine, dppb (1,4-bis(diphenylfosfino)butane), or dppf (diphenylphosphineferrocene), preferably triphenylphosphine.

When a palladium salt catalyst is used, preferably the molar ratio of the organic ligand to the catalyst approx. ranges from 2:1 to 4:1, preferably approx. from 2.8:1 to 3.2:1, in particular 3:1.

A basic agent can be an organic base, such as a straight or branched tertiary amine, typically triethylamine; or an inorganic base, such as an alkali metal, in particular potassium, sodium or cesium, carbonate; sodium acetate; an alkali metal, in particular sodium or potassium, hydroxide; potassium phosphate; or potassium hydrogen phosphate; preferably potassium carbonate or an alkali metal hydroxide, in particular potassium hydroxide.

The molar ratio of basic agent to compound of formula (**II**) approx. ranges from 4:1 to 10:1, preferably approx. from 5:1 to 8:1.

The reaction can be carried out in the presence of an organic solvent, or in a mixture of two or three organic solvents; or in a mixture of one, two or three thereof with the water. An organic solvent is typically an aromatic hydrocarbon, for example toluene, xylene; an ether, for example tetrahydrofuran, methyl-tetrahydrofuran, dioxane; an ester, for example ethyl acetate or butyl acetate; a dipolar aprotic solvent, for example dimethylformamide, dimethylacetamide, dimethylsulfoxide or N-methylpyrrolidone; or an alkanol, for example methanol, ethanol or isopropanol. The reaction is preferably carried out in a tetrahydrofuran-water or toluene-isopropanol-water mixture.

Compound **(III)** is preferably added in the solid form and in portions to the reaction mixture; or a solution thereof in one or more of the above mentioned solvents, optionally containing up to 70% of the basic agent amount, is added in portions, for example dropped, to the reaction mixture; in a time of about 2-8 hours, preferably about 3-6 hours, to minimize its degradation.

The reaction can be carried out at a temperature approx. ranging from 0°C to the reflux temperature of the reaction mixture, preferably approx. from 30°C to the reflux temperature, more preferably from 50 to 80°C.

According to a preferred aspect, the reaction can be carried out between a compound **(II),** wherein X is bromine, and a synthon **(III),** wherein M is a - B(OR₁OR₂) group wherein each of R₁ and R₂ is hydrogen; in the presence of palladium (II) acetate, triphenylphosphine, potassium hydroxide, in a tetrahydrofuran-water mixture or toluene-isopropanol-water mixture. More preferably, when in a compound of formula (II) the Z is the amide residue (S)-N-(1-carboxy-2-methylprop-1-yl)-N-pentanoylamino, then the solvent is a toluene-isopropanol-water mixture.

If desired, a compound of formula **(I)** can be converted to a salt thereof or *vice versa,* according to known methods.

Compounds of formula **(II)** wherein Z has the meanings a) and c), reported above, are known and can be prepared, as reported in J. Org. Chem. 1994, 59, 6391-6394, by alkylation of the respective heterocycle.

Compounds of formula **(II)** wherein X is as defined above, and is preferably a halogen atom, in particular bromine, and Z has the meanings b) and e) reported above, and the salts thereof, are novel compounds and are a further object of the invention. These compounds can be prepared analogously.

Compounds of formula **(II)** wherein Z has the meaning d) reported above, wherein leaving group X is nonafluorobutanesulfonyloxy, as well as their possible isomers, in particular the optical isomers, and the salts thereof, are novel compounds and are a further object of the invention.

A preferred example of a compound of formula **(II),** wherein Z has the meaning d), is (S)-2-[(4-nonafluorobutanesulfonyloxy-benzyl)-pentanoylamino]-3-methyl-butyric acid.

A compound of formula **(II)** wherein Z has the meaning d) and X is a leaving group as defined above, or a salt thereof, can be obtained by reaction of a compound of formula **(IV),** wherein X is as defined above, with a hydrogen donor.

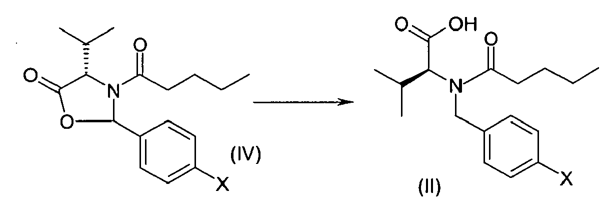

A hydrogen donor can be for example the triethylsilane/trifluoroacetic acid system; or an alkali or alkaline-earth borohydride, typically sodium or lithium borohydride; molecular hydrogen in the presence of a Pd, Pt or Ni catalyst; sodium, potassium, or ammonium formate, or an alkene or diene system, such as cyclohexene, cyclohexadiene, limonene. The hydrogen donor is preferably the triethylsilane/trifluoroacetic acid system or hydrogen in the presence of a Pd, Pt or Ni catalyst.

The reaction can be carried out in the presence of an organic solvent selected from, for example, those mentioned above, or in a chlorinated solvent, preferably in dichloromethane, and at a temperature approx. ranging from 0°C to the reflux temperature of the reaction mixture, preferably approx. from 20 to 50°C.

Alternatively, a compound of formula **(II),** wherein Z has the meaning d), or a salt thereof, can be obtained by the process reported in the following Scheme, wherein X is as defined above.

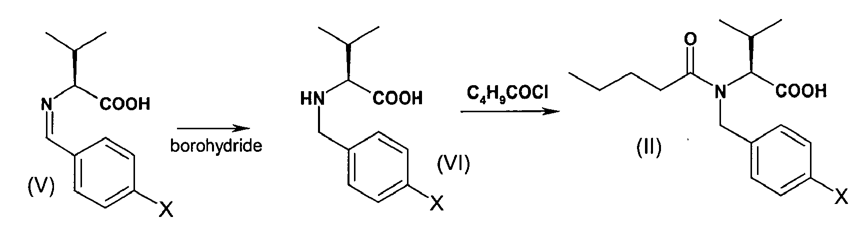

Said process comprises acylating a compound of formula **(VI)** with valeroyl chloride in the presence of a basic agent. The basic agent can be one of those described above for the reaction between a compound **(II)** and a compound **(III).** The basic agent is preferably triethylamine. The stoichiometric ratio of valeroyl chloride to compound **(VI)** can approx. range from 5:1 to 1:1, preferably approx. from 2:1 1 to 1:1. The stoichiometric ratio of basic agent to compound **(VI)** can approx. range from 10:1 to 2:1, preferably approx. from 5:1 1 to 2:1. The reaction can be carried out in water or in the presence of an organic solvent, selected from, for example, those described above for the reaction between a compound **(II)** and a compound **(III),** or mixtures of said solvents or mixtures thereof with water. The reaction is preferably carried out in water or in a mixture of an organic solvent with water, in particular water.

The reaction can be carried out at a temperature approx. ranging from -10°C to the reflux temperature of the mixture, typically approx. from 0 to 5°C.

If desired, the resulting compound of formula (II) wherein Z is the residue (S)-N-(1-carboxy-2-methylprop-1-yl)-N-pentanoylamino can be converted into a salt thereof or *vice versa,* according to known methods.

If desired compound of formula (II) or a salt thereof, thus obtained, can be further reacted with a synthon of formula (III) or a salt thereof, as defined above, for instance according to the process of the invention, to obtain a compound of formula (I), in which Z, being as defined above, is the residue (S)-N-(1-carboxy-2-methylprop-1-yl)-N-pentanoylamino, namely valsartan, or a salt thereof. A compound **(VI)** can be obtained by reaction of a compound **(V)** with an alkali or alkaline-earth borohydride, particularly sodium borohydride. The reaction can be carried out in an alkanol, as indicated above, or in mixtures of said alkanol with one of the organic solvents cited above. The reaction is preferably carried out in methanol.

Synthons of formula **(III)** are known or can be prepared with known methods, for example according to WO 05/014560.

The compounds of formula **(IV)** and **(V)** are known from EP 1 533 305 A1.

The following examples illustrate the invention.

### Example 1

### Synthesis of (S)-2-(4-bromo-benzyl)-amino-3-methyl-butyric acid; (VI)

45.0 g of sodium methoxide (30% w/w in methanol) are dropped into a suspension of 29.2 g of L-valine and 250 ml of methanol, under nitrogen. The mixture is left under stirring at room temperature to complete solution. The resulting clear solution is added with 47.1 g of p-bromobenzaldehyde and kept under stirring for 90 minutes. After that, 7.2 g of sodium boron hydride are added to the reaction mixture that is left under stirring for 1 hour, then concentrated under vacuum at a temperature of 40 - 50°C to obtain a very concentrated but still stirrable mass (about 120 - 150 ml), which is added with 400 g of water. Methanol is distilled off under vacuum. The reaction mixture is then acidified to pH 2 with 37% HC1 w/w, to precipitate the product, which is filtered with suction, washed with some water and dried under vacuum at 50°C to obtain 70 g of dry product.

### Example 2

### Synthesis of (S)-2-[(4-bromo-benzyl)-pentanoyl-amino]-3-methyl-butyric acid; (II)

A 1000 ml jacketed reactor, purged with nitrogen, is loaded with 70 g of (S)-2-(4-bromo-benzyl)-amino-3-methyl-butyric acid in 325 g of water and 108 g of triethylamine. The mixture is stirred at room temperature to completed solution, then cooled to -5°C and dropwise added with 32 g of valeroyl chloride, keeping this temperature. When the addition is over, the mixture is left to stand for 30 min, then warmed to 15°C and added with 180 ml of toluene. 60 ml of glacial acetic acid are then dropped into the stirred mixture to pH 5. Phases are separated, the lower one is discarded, and the other is added with 250 ml of water and 20 g of aqueous 50% w/w NaOH to pH 10. Phases are separated, the organic one is discarded, and the aqueous one is added first with 150 ml of heptane, then with 37% w/w HCl to pH 1. The resulting solid is left under stirring for 1 hour, then filtered with suction, and dried in static dryer at 50°C, thereby obtaining about 70 g of the title product, in an about 77% yield.

By proceeding analogously (S)-2-[(4-nonafluorobutanesulfonyloxybenzyl)-pentanoyl-amino]-3-methyl-butyric acid can be obtained.

### Example 3

### Synthesis of valsartan

An aqueous solution (120 ml) of potassium hydroxide (0.568 mol, 31.8 g) is added in succession with 2-[(4-bromo-benzyl)-pentanoyl-amino]-3-methyl-butyric acid (0.811 mol, 30.0 g), tetrahydrofuran (120 ml), triphenylphosphine (0.0121 mol, 3,2 g) and palladium acetate (0.00405 mol, 0.91 g). The reaction mixture is refluxed and added with 2-(2H-tetrazol-5-yl)-benzene-boronic acid (0.142 mol, 27.0 g) in portions in about 6 h. After completion of the addition, the mixture is left to react for 2h, then cooled to room temperature and the phases are separated. The organic phase is diluted with water (120 ml) and tetrahydrofuran is distilled off under reduced pressure. The remaining aqueous solution is acidified to pH 6.5 and washed with isopropyl acetate (60 ml). The aqueous phase is acidified to pH 2 and diluted with isopropyl acetate (60 ml), the diphasic solution is filtered to remove phenyltetrazol. Phases are separated and the organic phase is concentrated under reduced pressure, to obtain a thick oil that is crystallized from isopropyl acetate (90 ml) and heptane (150 ml). The resulting product is filtered, washed twice with a 1:1 isopropyl acetate/heptane mixture (30 ml), and dried in static dryer at 45°C to obtain 28.2 g of the title product (yield 80%).

Following the same procedure, starting from a compound **(II)** wherein X is bromine and Z a residue selected from:
2-butyl-4-chloro-5-hydroxymethyl-imidazol-1-yl;
2-ethoxy-3H-benzoimidazol-4-carboxylic acid;
2-butyl-1,3-diaza-spiro[4,4]non-1-en-4-on-3-yl; and
5-carboxy-4-( 1-hydroxy-1-methylethyl)-2-propyl-imidazo-1-yl,
losartan, candesartan, irbesartan and olmesartan can be respectively obtained.

### Example 4

### Synthesis of losartan potassium

An aqueous solution (8 ml) of potassium hydroxide (0.030 mol, 1.7 g) is added in succession with [3-(4-bromo-1-benzyl)-2-butyl-5-chloro-3H-imidazol-4-yl]-methanol (0.013 mol, 4.8 g), toluene (20 ml), isopropanol (5 ml), triphenylphosphine (0.0019 mol, 0.5 g) and palladium acetate (0.00067 mol, 0.15 g). The reaction mixture is heated to 70°C and a solution of 2-(2H-tetrazol-5-yl)-benzene-boronic acid (0.0135 mol, 2.56 g) and potassium hydroxide (1.9 g, 0.034 mol) in water (5 ml) is dropped therein in about 4h. After completion of the addition, the mixture is reacted for 1 h, then cooled to room temperature, diluted with water (20 ml) and acidified to pH 2. Phases are separated, the aqueous one is discarded and the organic phase is concentrated under reduced pressure. The resulting thick oil is diluted with methanol (30 ml) and added with 2 g of NaHCO₃. The mixture is heated to ebullition until effervescence ceases, then the residual solid is filtered off. After cooling to 20°C, 50 ml of methyl-t-butyl ether are added. The resulting precipitate is filtered and dried in static dryer at 45°C to obtain 4.5 g of the title product (75% yield).

Following the same procedure, starting from a suitable compound (**II**), candesartan, irbesartan, valsartan and olmesartan can be obtained.

## Claims

1. A process for the preparation of a compound having formula (I), or a salt thereof, 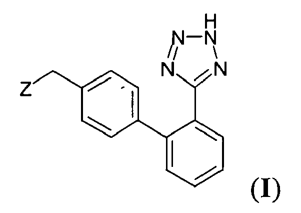 wherein
Z is an optionally substituted heterocycle containing at least one
nitrogen atom, or an open amide residue;
which comprises
reacting a compound of formula (**II**), or a salt thereof 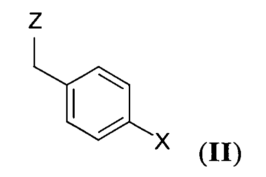 wherein
X is a leaving group and Z is as defined above;
with a synthon of formula (**III**), or a salt thereof 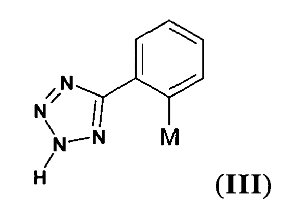 wherein
M is a -B(OR₁OR₂) group wherein
each of R₁ and R₂ is independently is hydrogen, C₁-C₈ alkyl, aryl, aryl-C₁-C₈ alkyl, or
R₁ and R₂, taken together, form a -(CH₂)ₘ-V-(CH₂)ₙ- group,
wherein
m and n, which can be the same or different, are 0 or 1, and
V is NR₃ or C(R₃)₂
wherein R₃ is hydrogen, C₁-C₈ alkyl, aryl or aryl-C₁-C₈ alkyl;or
M is a lithium or copper atom or a halogenated metal;
in the presence of a catalyst, an organic ligand and a basic agent and, if desired, converting a compound of formula (**I**) to a salt thereof and/or, if desired, converting a salt of a compound of formula (**I**) into its free acid; and wherein when, at the same time, Z is the amide residue (S)-N-(1-carboxy-2-methylprop-1-yl)-N-pentanoylamino, X is halogen or an -OSO₃R group, in which R is CF₃, tosyl, mesyl or F, M is a -B(OR₁OR₂) group wherein each of R₁ and R₂ is hydrogen, and the catalyst is a palladium compound, then the reaction is carried out in a solvent other than an aqueous or polar organic solvent, or a mixture of water and water-miscible solvent.

2. A process according to claim 1, wherein in a compound of formula (**II**) substituent Z is a residue selected from
a) 2-butyl-4-chloro-5-hydroxymethyl-imidazol-1-yl;
b) 2-ethoxy-3H-benzoimidazolo-4-carboxylic acid;
c) 2-butyl-1,3-diaza-spiro[4,4]non-1-en-4-on-3-yl;
d) (S)-N-(1-carboxy-2-methylprop-1-yl)-N-pentanoylamino; and
e) 5-carboxy-4-(1-hydroxy-1-methylethyl)-2-propyl-imidazo-1-yl.

3. A process according to claim 1, wherein the leaving group X is a halogen atom.

4. A process according to claim 1, wherein in a compound of formula (**III**) M is a -B(OR₁OR₂) group wherein each of R₁ and R₂ is independently hydrogen or C₁-C₄ alkyl.

5. A process according to claim 1, wherein the catalyst is a Pd, Pt or Ni salt.

6. A process according to claim 5, wherein the catalyst is a palladium **(II)** salt.

7. A process according to claim 1, wherein the organic ligand is a phosphine.

8. A process according to claim 7, wherein the organic ligand is triphenylphosphine.

9. A process according to claim 5, wherein the molar ratio of the organic ligand to the palladium salt catalyst approximately ranges from 2:1 to 4:1.

10. A process according to claim 9, wherein the molar ratio ranges approximately from 2.8:1 1 to 3.2:1.

11. A process according to claim 1, wherein the basic agent is an organic or inorganic base.

12. A process according to claim 11, wherein the basic agent is potassium carbonate or an alkali metal hydroxide.

13. A process according to claim 1, wherein the molar ratio of basic agent to compound of formula **(II)** approx. ranges from 4:1 to 10:1.

14. A process according to claim 1, wherein compound of formula **(III)** is added in portions to the reaction mixture either in the solid form or in the form of a solution, optionally containing up to 70% of the basic agent amount.

15. A process according to claim 1, wherein the reaction is carried out in the presence of an organic solvent, or in a mixture of two or three organic solvents; or in a mixture of one, two or three of them with water.

16. A process according to claim 15, wherein the reaction is carried out in a tetrahydrofuran-water or in a toluene-isopropanol-water mixture.

17. A process according to claim 15, wherein the reaction is carried out in a toluene-isopropanol-water mixture and the catalyst is a Pd salt.

18. A process according to claim 1, wherein in a compound **(II),** X is bromine, in a synthon of formula **(III),** M is a -B(OR₁OR₂) group wherein each of R₁ and R₂ is hydrogen; and the reaction is carried out in the presence of palladium (II) acetate, triphenylphosphine, potassium hydroxide, in a solvent chosen from a tetrahydrofuran-water mixture and toluene-isopropanol-water mixture.

19. A process according to claim 18, wherein in a compound of formula (II) the Z residue is (S)-N-(1-carboxy-2-methylprop-1-yl)-N-pentanoylamino and the solvent is a toluene-isopropanol-water mixture.

20. A compound of formula **(II),** or a salt thereof, 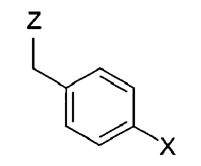 wherein X is a leaving group and Z is a residue
b) 2-ethoxy-3H-benzoimidazolo-4-carboxylic acid; or
e) 5-carboxy-4-(1-hydroxy-1-methylethyl)-2-propyl-imidazo-1-yl.

21. A compound of formula **(II)** according to claim 20, wherein X is a halogen atom.

22. A compound of formula (II), as defined in claim 1, which is (S)-2-[(4-nonafluorobutanesulfonyloxy-benzyl)-pentanoyl-amino]-3-methyl-butyric acid.

23. A process for the preparation of a compound of formula **(II),** or a salt thereof, in which Z is the residue (S)-N-(1-carboxy-2-methylprop-1-yl)-N-pentanoylamino, having the following furmula 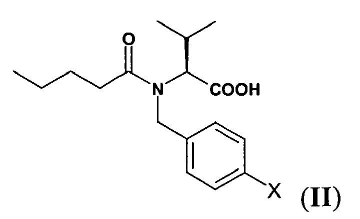 wherein X is a leaving group; comprising reacting a compound of formula **(IV)** 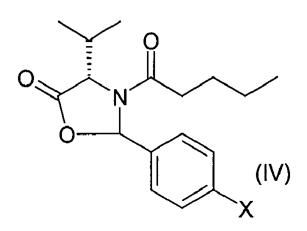 wherein X is as defined above, with a hydrogen donor; or
the acylation of a compound of formula **(VI)** 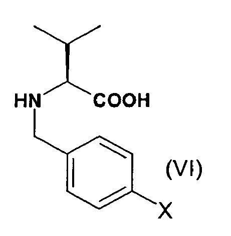 wherein X is as defined above,
with valeroyl chloride in the presence of a basic agent, in water or a mixture of an organic solvent with water and, if desired, converting the resulting compound of formula **(II)** to a salt thereof and/or, if desired, converting a salt of said compound into its free acid.

24. A process according to claim 23, further comprising reacting a thus obtained compound of formula **(II),** or a salt thereof, with a synthon of formula (III), as defined in claim 1, to obtain of a compound of formula (I), as defined in claim 1, or a salt thereof, in which Z is the residue (S)-N-(1-carboxy-2-methylprop-1-yl)-N-pentanoylamino.
